# EUROPEAN PATENT SPECIFICATION

(11) **EP 3 716 853 B1**
(45) Date of publication and mention of the grant of the patent: **10.01.2024**
(21) Application number: 18811811.1
(22) Date of filing: 30.11.2018
(51) Int. Cl.: A61B 6/03, A61B 6/00, G01T 1/29

(54) **POSITRON EMISSION TOMOGRAPHY (PET) SYSTEMS WITH TRANSFORMABLE TASK-OPTIMAL GEOMETRY**
SYSTEME ZUR POSITRONENEMISSIONSTOMOGRAPHIE (PET) MIT TRANSFORMIERBARER AUFTRAGSOPTIMALER GEOMETRIE
SYSTÈMES DE TOMOGRAPHIE PAR ÉMISSION DE POSITRONS (TEP) À GÉOMÉTRIE OPTIMALE DE TÂCHE TRANSFORMABLE

(30) Priority: 01.12.2017 US 201762593296 P
(43) Date of publication of application: 07.10.2020
(73) Proprietor: Koninklijke Philips N.V., 5656 AG Eindhoven (NL)
(72) Inventor: BAI, Chuanyong, 5656 AE Eindhoven (NL); ANDREYEV, Andriy, 5656 AE Eindhoven (NL); ZHU, Yang-Ming, 5656 AE Eindhoven (NL); ZHANG, Bin, 5656 AE Eindhoven (NL); TUNG, Chi-Hua, 5656 AE Eindhoven (NL); MCKNIGHT, Douglas, 5656 AE Eindhoven (NL)
(74) Representative: Philips Intellectual Property & Standards
(86) International application number: PCT/EP2018/083148
(87) International publication number: WO 2019/106150

(56) References cited:
- EP-A2- 1 860 465
- WO-A1-2015/124023
- US-A- 5 998 792
- US-A1- 2002 148 970
- US-A1- 2015 119 704

## Description

### FIELD

The following relates generally to the medical imaging arts, positron emission tomography (PET) imaging arts, and related arts.

### BACKGROUND

Positron emission tomography (PET) scanners typically include a cylindrical bore-type housing supporting several PET detector rings for detecting 511 keV gamma rays. These PET scanners have a field of view (FOV) with fixed axial and radial dimensions. Commercial PET scanners have been developed with increasingly large bore diameters, so as to accommodate larger patients. However, such designs increase the cost as the number of detectors increases with bore diameter. In the axial direction, the usual solution is to employ multi-stage imaging, in which the patient is stepped through the bore and imaged at several positions that overlap in the axial direction. These individual PET images are then "stitched" together at the axial overlaps to form a whole-body image (or other image with extended axial extent). This solution has disadvantages including potential for error at the stitched overlap regions, and increased imaging session time required to acquire the multiple images along the axial direction. It is also not possible to perform certain continuous acquisition dynamic studies with a single bed position.

To increase the axial FOV (AFOV) without a concomitant increase in the number of PET detector modules (and hence increased cost), it is known to provide gaps between adjacent PET detector rings. The axial FOV of the system is approximately the sum of the axial dimension of the rings and the gaps between the rings. In another approach, the detectors can be sparsely populated around the circumference of each detector ring, so that each ring has a reduced number of PET detector modules so that more rings can be added to increase the axial FOV. Zhang et al., "PET System With Crystal or Detector Unit Spacing", WO 2015/019312 A1 discloses embodiments of "sparse" designs, including embodiments in which the spacing(s) between adjacent detector rings can be adjusted for specific imaging tasks.

In a variant approach (*see* Gagnon et al., "Positron emission tomography system with hybrid detection geometries and sampling", U.S. Pat. No. 8,558,181), an adjustable axial FOV is provided. Detector bars are arranged parallel to the axial axis of the bore of the PET scanner and populated along a circle surrounding the patient. The bars can shift in the axial direction relative to each other by the desired amount to achieve the desired axial FOV while keeping a central axial region into which all the bars extend, providing full detector coverage for this central axial region. The regions/organs of interest will be aligned with the central axial region to optimize the imaging for such regions/organs.

In further previous approaches (*see* Gagnon et al., "Modular multi-geometry PET system", U.S. Pat. No. 8,378,305), a dual detector PET system includes two detector sets to image different portions of a patient and an adjustable detector ring having one set of detectors that can move in and out radially to form different size of transaxial rings to image patient of different sizes, while the other set of detectors can acquire data simultaneously if desired.

Further prior art can be found in EP 1860465 A2 and US 2015/119704 A1.

The following discloses new and improved systems and methods.

### SUMMARY

According to the present invention, a PET imaging device is presented as defined in the claims

One advantage resides in providing a positron emission tomography (PET) imaging device with radiation detectors or detector modules being individually controllable in multiple directions (e.g. axially and/or radially and/or tangentially) to configure the PET scanner for a particular patient and/or task.

Another advantage resides in providing an imaging device with movable radiation detectors to increase or decrease an axial field of view of the imaging device with reduced loss of data coverage in the increased FOV configuration by way of oscillating the detector modules axially and/or tangentially to provide oversampling.

Another advantage resides in providing an imaging device with an increased axial field of view and a reduced number of detectors.

Another advantage resides in providing an imaging device with movable detectors that conform to a subject geometry of a patient.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURES 1-3 diagrammatically show an image reconstruction system according to one aspect.
FIGURES 4 and 5 show exemplary flow chart operations of the system of FIGURES 1-3;
FIGURES 6-11 show different example configurations of the system of FIGURES 1-3.

### DETAILED DESCRIPTION

The following proposes a configurable PET scanner in which the PET detector positions can be optimized for a particular imaging task. In one embodiment, the PET detector modules are mounted on racks, which are tracks that allow the PET detector modules to be moved longitudinally (i.e. axially, i.e. in the z-direction) along the length of the rack. Further, each PET module is mounted to the rack via a robotic telescoping arm to provide for movement in the radial direction. Still further, each rack may be moved along the tangential (i.e. angular) direction. With these three degrees of robotic freedom, a wide range of PET scanner configurations can be achieved. For example, a larger axial FOV can be achieved by allowing for larger gaps between rings of PET modules via movement of the PET modules in the axial direction. In another approach, sets of PET modules in different angular intervals can be relatively offset to provide increased axial FOV.

The flexibility also allows for non-uniform PET module positioning, for example in a cardiac scan the density of rings near to the heart location can be increased (up to having no gap between adjacent rings) relative to the peripheral rings. In this regard, it is contemplated to mount radiation shields between adjacent PET modules on independent robotic telescoping arms, thus allowing these shields to be withdrawn from between central rings to maximize ring density in the central region.

In some embodiments, the PET modules can be moved during a PET imaging data acquisition. For example, if a larger axial FOV is achieved by way of spacing apart the neighboring PET rings, those rings could be moved during the acquisition so that there are no axial gaps in the final collected data set. A similar concept is "oversampling", in which detectors are moved back and forth during the acquisition to increase axial detector resolution; similar angular back and forth movement could be used to increase detector resolution in the tangential direction.

In some embodiments, different PET modules may comprise different detector types, e.g. a mix of TOF-PET modules and non-TOF-PET modules, and the configurability of the PET scanner leveraged to optimally position the mixture of PET module types. In a similar fashion, PET modules with larger density of dead pixels could be compensated by moving other PET modules with fewer dead pixels close by to compensate for the dead pixels.

In some embodiments, in any PET scanner configuration for which the acquisition setup is less than ideal, e.g. with gaps between rings or so forth, it may be possible to acquire phantom data for the PET scanner configured both with such non-ideality and in a more ideal configuration (e.g. without gaps), and deep learning used to train a transform to adjust an image acquired using the non-ideal configuration to more closely mimic the ideal configuration.

In other embodiments, the disclosed PET system may require different or additional configuration robotics as compared with the illustrative rack arrangement. For example, in the case of a breast examination, detectors could additionally be provided with a tilt robotic adjustment, and two PET modules located between the breasts can be tilted to face the opposing breasts, thereby providing PET counts in those directions. Advantageously, with such an arrangement it may be possible to image both breasts simultaneously, whereas current PET breast imagers use a single cup and image one breast at a time.

In addition to appropriate robotic manipulators, the robotic controller tracks the current location (and angulation, in the case of tilting PET modules) of each PET detector module in order to accurately record the line of response (LOR) spatial trajectories. In one approach, a detector is defined to have a nominal position (z, *,r, θ)* where z is the default axial position, r is the default radial position, and *θ* is the default tangential (i.e. angular) position. This is updated in a particular PET module to a value *(z* + *Δz, r* + *Δr, θ* + Δθ) where *Δz* is the axial shift of the PET module along the rack, *Δr* is the radial shift of the PET module, and Δθ is the tangential (angular) shift of the supporting rack. The LOR is then conventionally defined given the positions of the two involved detectors in three-dimensional space. Additionally, the sensitivity matrix used in PET image reconstruction may need to be adjusted, especially when the PET detector modules are configured to a non-uniform arrangement which may, for example, increase sensitivity near the center of the scanner versus the axial periphery by having a higher density of detector rings positioned at/near scanner center.

The choice of PET scanner configuration for a particular imaging task can be variously chosen. In the simplest approach, the configuration is chosen manually, e.g. adding annular gaps between neighboring PET rings (or axial offsets between angularly neighboring racks) sized to achieve a desired axial FOV, setting radial positions of the PET modules to a minimum practical radial position for a patient of a certain girth, or so forth. In a more complex approach, it is contemplated to use an inverse optimization algorithm similar to inverse planning optimization employed in Intensity-modulated radiation therapy (IMRT) planning. In inverse IMRT planning, the radiation sources configuration is chosen, the resulting dose (or fluence) distribution in the target modeled taking into account radiation absorption using an attenuation map, and the sources configuration updated to improve matching between the modeled dose or fluence distribution and dose optimization goals. In similar fashion, the PET modules configuration can be chosen, the resulting counts distribution in the target modeled taking into account radiation absorption using an attenuation map, and the PET modules configuration updated to improve matching between the modeled counts distribution and dose optimization goals.

In imaging tasks employing multiple bed positions, the disclosed PET scanner configuration could in general be different for each bed position.

The disclosed system contains multiple racks distributed along the frame of the gantry that surrounds the patient (and bed or pallet on which the patient lies). For a simple implementation of this idea, the racks are parallel to each other and they are all parallel to the axial axis of the gantry. The racks are sufficiently long for the maximal AFOV to be achieved. In other examples, each rack can have multiple segmented pieces, and the segments can have offset in the plane perpendicular to the racks; racks or segments of racks can be in different orientations relative to each other and they don't have to be parallel to each other, etc.

The disclosed detectors can be designed as plug-in components with associated assembly peripherals. For example, the detectors can be plugged into the racks and their positions on the racks can be independently controlled and is programmable by the system. The system can include a mechanism to allow the detector to move closer to or retract from an object of interest either by moving the racks or rack segments or by moving the PET detector modules. The system can also allow the detector to reorient to point to an object of interest. The PET detector modules includes a mechanism to move and reorient the detector as controlled by the system. In some embodiments, the PET detector modules can include a collision detection and prevention mechanism. For example, one or more pressure sensors (not shown) can be disposed at corners of the PET detector modules. One or more electronic processors can analyze pressure signals obtained by the pressure sensors to determine if a collision between PET detector modules (or between one of the PET detectors and a patient to be imaged) is occurring. The processors can then control the PET detector modules to move away from each other avoid a collision.

For example, if each rack includes five detectors, and the detectors are pushed together to align all the detectors on all of the racks, the system configuration is the same as a conventional PET system with about 16 cm AFOV (assuming the detector assembly has dimension of 3.2 cm x 3.2 cm).

In some examples, since the detectors can be independently controlled and their locations on the racks are known, the neighboring PET detector modules 18 can be positioned so that there is a pre-determined gap along the rack (along an axial direction).

In other examples, all the detectors on one rack can be moved together, but the detectors on different racks can be moved by different amounts.

The previous two examples can be implemented in the disclosed PET system to increase the AFOV of image acquisition. These two example embodiments can be implemented to further increase the AFOV of the disclosed PET system.

Since the pre-determined gaps between the detectors on the racks is programmable, the disclosed PET system has the flexibility of adjusting the gap as desired. For imaging of small organs/objects, the gap can be set to zero so that maximal sensitivity can be obtained using the same number of detector areas.

In some examples, the number of detectors on each rack can be different. For example, one third of the racks of the gantry can have, for example, seven detectors (-22.4 cm), and the remaining portion of the racks can have, for example, four detectors each (-12.8 cm). The racks with seven detectors can provide an effective AFOV of 22.4 cm. The detectors on the four-detector racks can be shifted according to the intended application for optimal performance. The total number of detector area is the same as a system with five detectors on each rack (or a system with five fully populated rings), the effective AFOV of which, however, is increased from 16.4 cm to 22.4 cm.

The configurable design of the disclosed PET system can allow the manufacturing of low-cost, high-performing, and scalable systems. For example, if each rack has three detectors, it is equivalent to a three-ring system with AFOV of 9.6 cm. Using the disclosed configurable design, the effective AFOV can be extended to that of five virtual rings, i.e., 16 cm.

The three-ring system described above can be easily upgraded to a four-ring, a five-ring system, etc. by adding one or two detectors to each rack. This flexibility will significantly help customers with different upgrading needs.

The flexibility of extending the AFOV allows for a lot dynamic studies to be performed. In contrast, such studies cannot be done on the conventional systems with the same detector area because the effective AFOV is too small.

The position of the detectors can be controlled individually and optimized collectively for the intended applications using an optimization program (similar to that in radiation therapy in which the multi-leaf collimators openings, delivery length at each angle, etc. are optimized).

Since the detectors are designed as swappable plug-in components, they can be shared among multiple systems as needed. For example, one or more detectors can (temporarily) be removed from an available three-ring system and use them on another three-ring system to achieve maximum effectiveness of a six-ring system. If the other system is a combined PET/CT system, removal of the PET detector modules **18** from the system does not impact the performance of the CT part of the system.

The detectors can be turned or rotated to point to organs of interest and or move to and from the organs of interest to improve the sensitivity, reduce background activity impact, and improve the quality of the acquired data.

When detectors on neighboring racks are shifted relatively in a predefined pattern, axial oversampling of the disclosed PET system can be achieved.

The disclosed detector configuration can change during the scan via an optimization program. For example, for a multi-frame whole-body scan using step-and-shoot bed motion, the detectors can be positioned differently at the head, head/neck frames, torso, and lower body acquisition frame. This potentially reduces the total acquisition time and improve the clinical workflow and patient throughput.

The disclosed racks are not necessarily implemented as racks in the system design if other alternatives are desired. For example, a two-dimensional (2D) surface surrounding the patient can be designed as the base for detectors to be mounted/plugged. The position of the detectors on a 2D surface can be programmable. The detectors can also include the mechanism to reorient or move toward or away from the patient.

In some examples, the disclosed PET system can be transformed into a mammography PET scanner, forming the rings of PET detector modules **18** around patients breasts, with few extra detectors positioned to the sides and to the back of the patient for extra projection views to allow for complete tomographic data.

In other examples, the disclosed PET system can be transformed into brain PET scanner by forming the rings around patient head.

In further examples, the disclosed PET system can also be reconfigured into preclinical small animal scanner.

In some example embodiments, an angle of a detector can be optimized by positioning the detectors closer to the patient's body. To do so, the thickness of the detector crystals can be reduced in order to minimize the effect of depth-of-interaction (DOI). This potentially reduces the cost of goods for the production while the effective sensitivity of the PET camera would still be large due to optimized solid angle.

In other example embodiments, the detectors can have different configurations, or can be positioned closer to the patient with a smaller crystal size, for optimization optimized for higher spatial resolution.

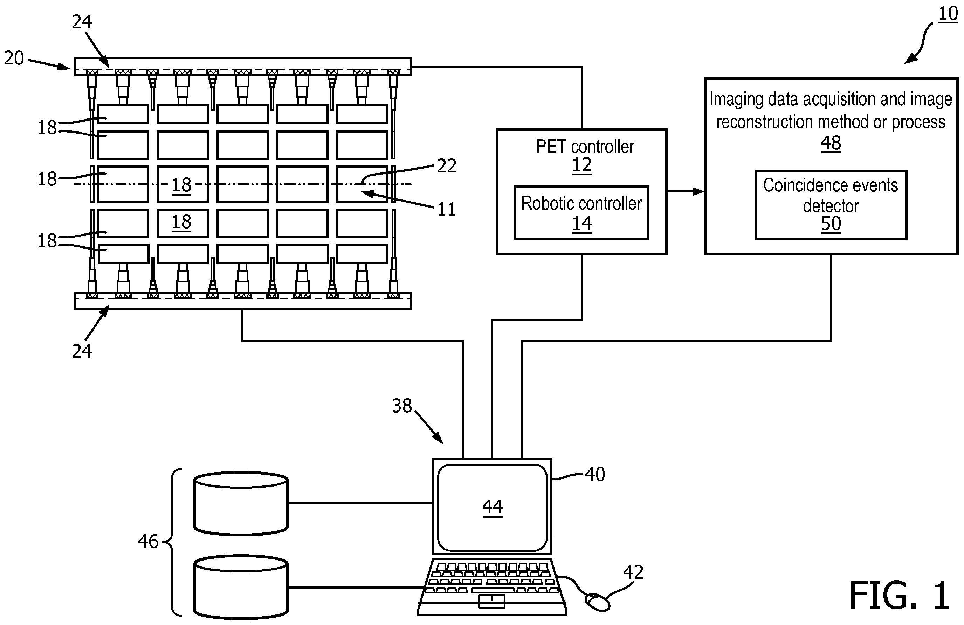

With reference to FIGURE 1, an illustrative positron emission tomography (PET) imaging system or device **10** receives a patient (not shown) into an examination region **11** for PET imaging. Although the imaging system **10** is described herein as a PET scanner, the imaging system can be any other suitable imaging modality (e.g., a gamma camera for a single photon emission computed tomography (SPECT) imaging device, a hybrid SPECT/PET imaging device, and so forth). The PET scanner **10** is controlled by a PET controller **12**, e.g. a computer or other electronic device including a microprocessor, microcontroller, or the like. As will be described, the PET scanner **10** employs a robotic gantry that is controlled by a robotic controller **14**. The PET scanner **10** is shown in FIGURE 1 in side-sectional view, and is seen to include a plurality of PET detector modules **18** supported by a robotic gantry **20** which in this illustrative embodiment includes a plurality of supporting racks **24**. Each PET detector module **18** includes an array of radiation detector pixels comprising suitable radiation detector devices (details not shown), such as scintillator crystals of a material that absorbs 511 keV gamma rays and generates a scintillation with each 511 keV absorption coupled with photomultiplier tube (PMT), digital or analog silicon photomultiplier (SiPM), or other detectors arranged to detect the scintillations generated in the scintillator crystals. The detailed configuration may be various, e.g. a one-to-one arrangement in which each detector pixel comprises a corresponding scintillator crystal and SiPM or other detector, or a distributed arrangement such as a large area scintillator crystal optically coupled with a plurality of PMTs, SiPMs, or so forth and employing Anger logic to localize each 511 keV detection event, or so forth.

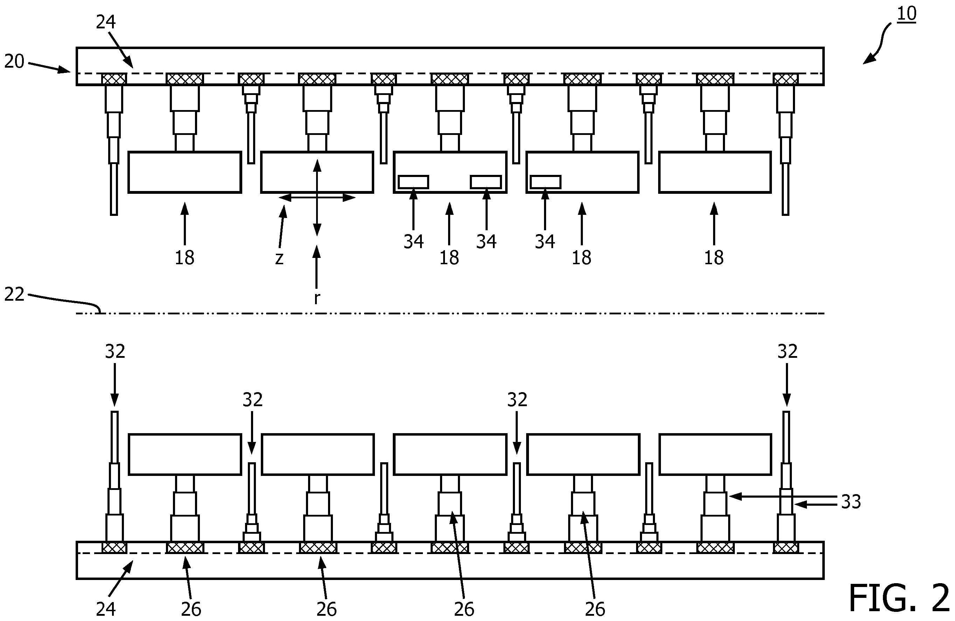

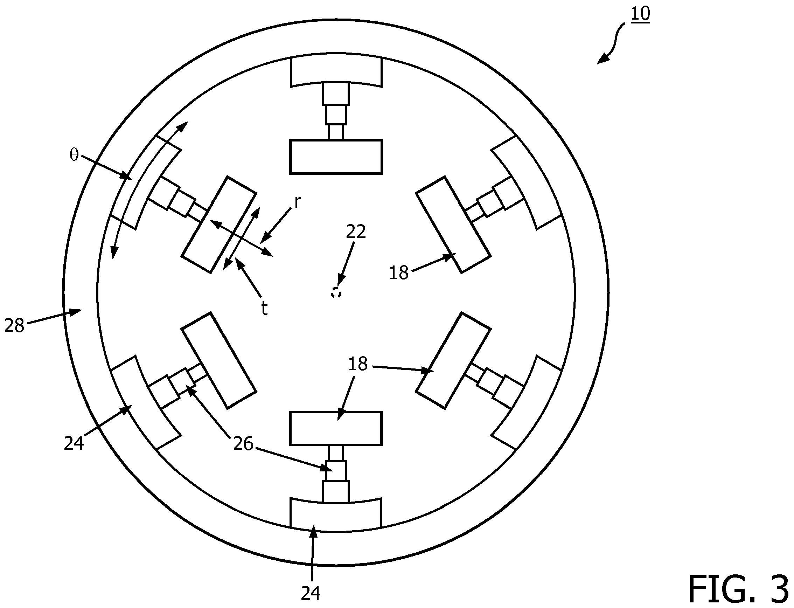

FIGURES 2 and 3 illustratively show the PET imaging system **10** in more detail. With continuing reference to FIGURE 1, and referring now to FIGURES 2 and 3, the PET imaging device **10** includes a plurality of PET detector modules **18** arranged to obtain PET imaging data of a patient in the examination region **16**. In some examples, the plurality of PET detector modules **18** can be identical to each other. In other examples, at least one of the PET detector modules **18** is different from another one of the PET detector modules according to: a material used to construct the radiation detectors of the PET detector module, one of the PET detector modules comprising time-of-flight detectors and another of the PET detector modules comprising non-time of flight radiation detectors, one of the PET detector modules includes time-of-flight PET detector modules having a different time-of flight-resolution than another one of the PET detector modules comprising time-of-flight PET detector modules; one of the PET detector modules including crystals of at least one of a different size and length than crystals of another one of the PET detector modules; and/or so forth.

A robotic gantry **20** is operatively connected to the plurality of PET detector modules **18**. The robotic gantry **20** configured to control a position of each PET detector module **18** along an axial axis **z** and/or a radial axis **r** (FIGURE 2) and/or a tangential axis **θ** (FIGURE 3) of the corresponding radiation detector. In some embodiments, the robotic gantry **20** is configured to independently control a position of each PET detector module **18** along two or more of the axial axis **z,** the radial axis **r**, and the a tangential axis **θ** of the corresponding radiation detector. Note that each PET detector module **18** comprises a one- or two-dimensional array of PET detector pixels supported on a common substrate or housing to move together as a unit. However, the robotic gantry **20** operates to move the PET detector modules **18**, or at least groups of the PET detector modules **18**, independently of one another, thereby permitting the plurality of PET detector modules **18** to be arranged in any of a wide range of different configurations.

As shown in FIGURE 2, the PET detector modules **18** of the PET imaging system **10** are arranged around a bore that, in the illustrative example, is a horizontal cylindrical bore having a defined bore axis **22**, which is parallel to the axial axis z of the PET detector modules **18**. (Note that in FIGURE 2 unlike FIGURE 1, the view of a section of only the upper and lower racks intersected by the section plane, so as to more clearly illustrate two representative racks). As seen in the full sectional view of FIGURE 1, a plurality of racks **24** are disposed around the bore axis **22**. Each PET detector module **18** is mounted to one of the racks **24**. Each rack **24** is oriented parallel with the bore axis **22**. Each PET detector module **18** is robotically movable in the axial direction (i.e. parallel with the bore axis **22**, or equivalently along the axial axis **z** of the PET detector module **18**) along the rack **24** supporting the PET detector module **18**. As shown in FIGURE 2, upper and lower racks **24** (and supported PET detector modules **18**) are shown on opposing sides of the bore axis **22** (the racks are mirror images of each other, but for clarity, some reference characters are included only for the "top" rack and other reference characters are included only for the "bottom" rack).

With continuing reference to FIGURES 1 and 2, and now referring to FIGURE 3 which shows an end view of the PET scanner **10**, a telescoping arm **26** is connected to, and supports, each PET detector module **18**. The telescoping arms **26** are operable to move the supported PET detector modules **18** along the radial axis **r** of the radiation detector, i.e. toward or away from the imaging subject disposed in the bore of the PET scanner **10** (or, equivalently, toward or away from the bore axis **22**).

As seen in FIGURE 3, the robotic gantry **20** further includes a plurality of rack support arcs or rings **28** each at least partially encircling the bore **22** of the imaging device **10**. In the end view of FIGURE 3, only one rack support ring **28** is visible, but typically multiple such support rings **28** are provided, e.g. one at each of the two opposite ends of the racks **24** and optionally one or more additional intermediate rack support rings in-between to provide additional support. In another contemplated variant, a single rack support ring may be provided which extends the full axial length of the PET scanner **10**, so that this single rack support ring is a cylinder axially coextensive with the racks **28**. The one or more rack support arcs or rings **28** include robotic links operable to move each rack **24** along a tangential axis θ transverse to the rack (see FIGURE 3), thereby moving the connected PET detector modules **18** along the tangential direction **t** (see FIGURE 3).

As labeled in FIGURE 2, the PET imaging device **10** also optionally includes a plurality of radiation shields **32** disposed in gaps between axially neighboring PET detector modules **18**. While not illustrated, it is similarly contemplated to include radiation shields disposed in gaps between tangentially neighboring PET detector modules **18**. The robotic gantry **20** is operatively connected to the radiation shields **32** by telescoping arms **33** to selectively extend or retract individual radiation shields **32**. For example, as shown in FIGURE 2, the pair of radiation shields **32** disposed at the ends of the robotic gantry **20** are extended past the PET detector modules **18** to provide radiation shielding, e.g. to reduce the detection of spurious out-of-axial FOV radiation, while the radiation shields **32** disposed between radiation detectors are retracted such that the PET detector modules **18** extend past the radiation shields.

In some embodiments, as shown in FIGURE 2, the PET detector modules **18** can include a collision detection and prevention mechanism including one or more pressure sensors **34**. For example, one or more pressure sensors **34** can be disposed at corners of the PET detector modules **18**. The PET controller **12** can analyze pressure signals obtained by the pressure sensors **34** to determine if a collision between neighboring PET detector modules (or between one of the PET detector modules and a patient to be imaged) is occurring. The PET controller **12** can then control the PET detector modules **18** to move away from each other avoid a collision.

Referring back to FIGURE 1, the robotic controller **14** comprises an electronic processor programmed to: determine a desired change in position along at least one of the axial axis **z**, the radial axis **r**, and the tangential axis **θ** of the corresponding PET detector modules **18**; and move the corresponding radiation detector along the determined change. A computer or workstation or other electronic data processing device **38** with typical components, such as at least one electronic processor **40**, at least one user input device (e.g., a mouse, a keyboard, a trackball, and/or the like) **42**, and a display device **44**, enables a radiologist, technician, or other medical personnel to interact with the PET controller **12** to operate the PET imaging device **10** to perform PET imaging data acquisition.

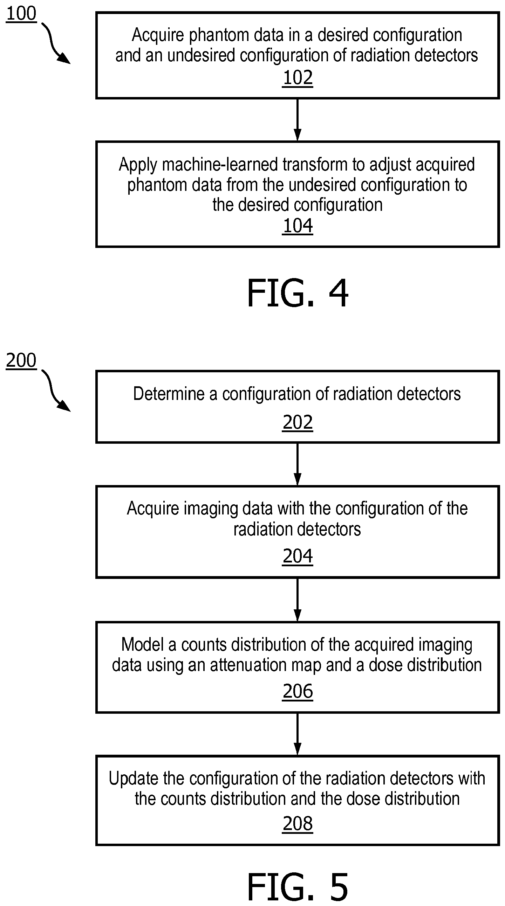

The at least one electronic processor **12**, **14**, **40** is operatively connected with one or more non-transitory storage media **46** (such as a magnetic disk, RAID, or other magnetic storage medium; a solid state drive, flash drive, electronically erasable read-only memory (EEROM) or other electronic memory; an optical disk or other optical storage; various combinations thereof; or so forth) which stores instructions which are readable and executable by the at least one electronic processor **12**, **14, 40** to perform operations disclosed herein such as performing a detector configuration update method or process **100**, **200** (see FIGURES 4 and 5) to configure the PET imaging device **10** for a received imaging subject geometry and/or for a received imaging task, and to perform an imaging data acquisition and image reconstruction process **48** which includes detecting coincidence events each comprising a pair of 511 keV detection events detected by PET detector modules **18** within a coincidence time window, and reconstructing the coincidence events to generate a reconstructed PET image. The image reconstruction may employ any suitable image reconstruction algorithm, e.g. maximum likelihood-expectation maximization (MLEM), ordered subsets expectation maximization (OSEM), or so forth, and may incorporate scatter correction, edge preserving regularization, and/or other techniques for enhancing image quality as are known in the art. Optionally, if the PET detector modules **18** include sufficiently fast PET detector modules to provide time-of-flight (TOF) localization along the lines of response (LORs), then the image reconstruction may leverage the TOF information in the image reconstruction.

With reference to FIGURE 4, an illustrative embodiment of a detector configuration update method **100** is diagrammatically shown as a flowchart. At **102**, the PET detector modules **18** are configured, or controlled by the at least one electronic processor (i.e. robotic controller **14**), to acquire phantom data of a subject in both a desired configuration and an undesired configuration of the radiation detectors. At **104**, the at least one electronic processor **40** is programmed to apply a machine-learned transform to the acquired phantom or patient data to adjust the PET detector modules **18** from the undesired configuration to the desired configuration of the PET detector modules **18**.

With reference to FIGURE 5, another illustrative embodiment of the radiation detector configuration update method **200** is diagrammatically shown as a flowchart. At **202**, the at least one electronic processor **40** is programmed to determine a configuration of the PET detector modules **18**. In some examples, the configuration of the PET detector modules **18** is determined for a received imaging subject geometry (e.g., one or more breasts). In some examples, the at least one electronic processor **40** is programmed to determine the detector configuration including axial positions of the PET detector modules **18** to encompass the received imaging subject geometry radial positions of the radiation detectors determined based on a girth of the received imaging subject geometry. In other examples, the at least one electronic processor **40** is programmed to determine the detector configuration comprising positioning of the PET detector modules **18** conformably with at least one surface of the received imaging subject geometry.

At **204**, the at least one electronic processor **40** is programmed to acquire imaging data with the configuration of the PET detector modules **18**. To do so, the at least one electronic processor **40** is programmed to operate the robotic gantry **20** to arrange the plurality of PET detector modules **18** in the determined detector configuration. With the plurality of PET detector modules **18** arranged in the determined detector configuration, the at least one electronic processor **12, 14, 40** is programmed to control the robotic gantry **20** to acquire imaging data using the PET detector modules and reconstruct the imaging data to generate a reconstructed image. In some examples, the at least one electronic processor **12, 14, 40** is further programmed to, during the acquisition of imaging data, operate the robotic gantry **20** to oscillate the PET detector modules **18** in at least one of the axial direction and the tangential directions, so as to perform oversampling. This can be useful if the configuration spaces the detector modules apart with gaps between the detector modules in order to cover a larger FOV - the oversampling can reduce the impact of the gaps on the completeness of the acquired imaging data set. In other examples, the PET detector modules **18** are disposed in a predefined range along at least one of the two directions (z, r, and θ). For oversampling operations, the PET detector modules **18** can be controlled by the robotic gantry **20** to move the PET detector modules to a different location, either continuously or in multiple steps for data acquisition. When a scan is done, PET detector modules **18** can resume an original position.

The illustrative robotic gantry **20** of FIGURES 1-3 is an illustrative example. Different and/or additional configuration robotics are also contemplated. It will be appreciated that not all three of the axial (z), radial (r), and tangential (t) degrees of freedom may be provided. For example, a robotic gantry providing the axial (z) and radial (r) degrees of freedom, but not the tangential (t) degree of freedom, can be useful in accommodating patients of different heights (corresponding to axial "length" when the patient is lying in a prone or supine position along the bore axis **22**) and different girths.

As another example, a robotic gantry providing the axial (z) and tangential (θ) degrees of freedom, but not the radial (r) degree of freedom, can be useful in accommodating patients of different heights and also employing fewer PET detector modules by providing for gaps between adjacent detector modules along the circumferential direction.

As another example, to implement a breast examination with conformal placement of PET detector modules around both left and right breasts, the robotics for positioning the PET detector modules could optionally be provided with a tilt robotic adjustment (not shown). With this additional robotic degree of freedom, two PET modules can be placed between the breasts, with one tilted to face the left breast and the other tilted to face the right breast, thereby providing PET counts in those directions. Advantageously, with such an arrangement imaging data can be collected for both breasts simultaneously.

In addition to appropriate robotic manipulators such as those described with reference to FIGURES 1-3 and optionally including the tilt mentioned above, the robotic controller **14** tracks the current location (and angulation, in the case of tilting PET detector modules) of each PET detector module **18** in order to accurately record the line of response (LOR) spatial trajectories of coincidence events. In one approach, a PET detector module is defined to have a default position and a given detector on that module then has a nominal position (*z*, *r*, *θ*) where z is the default axial position, r is the default radial position, and *θ* is the default tangential (i.e. angular) position of the detector. This is updated in a particular PET detector module to a value (*z* + Δ*z*, *r* + Δ*r*, *θ* + Δ*θ*) where Δ*z* is the axial shift of the PET detector module along the rack **24**, Δ*r* is the radial shift of the PET detector module achieved by the telescoping arm **26**, and Δ*θ* is the tangential (angular) shift of the rack **24** supporting the PET detector module. More generally, the location of each 511 keV detection event in PET detector module coordinates is transformed to a location in PET imaging device coordinates by shifting the location of the 511 keV detection event in PET detector module coordinates in accord with the position of the PET detector module along the axial axis (z), the radial axis (r), and the tangential axis (t) of the PET detector module containing that radiation detector. The LOR is then defined as connecting the locations of the pair of 511 keV detection events in PET imaging device coordinates. Additionally, the sensitivity matrix used in PET image reconstruction **48** may need to be adjusted, especially when the PET detector modules are configured to a non-uniform arrangement which may, for example, increase sensitivity near the center of the PET scanner **10** versus the axial periphery by having a higher density of PET detector modules positioned at or near scanner center.

In some embodiments, the at least one electronic processor **40** is programmed to repeat the determination of the detector configuration, the operating of the robotic gantry **20** to arrange the plurality of PET detector modules **18** in the determined detector configuration, and the acquisition of imaging data for a plurality of bed positions to perform multi-station imaging.

At **206**, the at least one electronic processor **40** is programmed to model a counts distribution of the acquired imaging data using an attenuation map and a dose distribution.

At **208**, the at least one electronic processor **40** is programmed to update the configuration of the PET detector modules **18** with the counts distribution and the dose distribution.

### EXAMPLES

The PET detector modules **18** are configurable in many suitable desired configurations. For example, the PET detector modules **18** can be configured as tiles, and designed as plug-in components. The PET detector modules **18** can be plugged into the racks **24** to face the patient, and can also be move to and from the patient and reorient to the regions of interest for optimized imaging.

The imaging system **10** can include optimization software to compute the optimal position/orientation of each PET detector module **18** according to the imaging task. For example, for a system with PET detector modules **18** equivalent to a conventional five-ring system with AFOV of 16.4 cm, if the imaging task needs an effective AFOV greater than 16.4 cm, the system can program AFOV extension and move the PET detector modules **18** accordingly to achieve the desired AFOV.

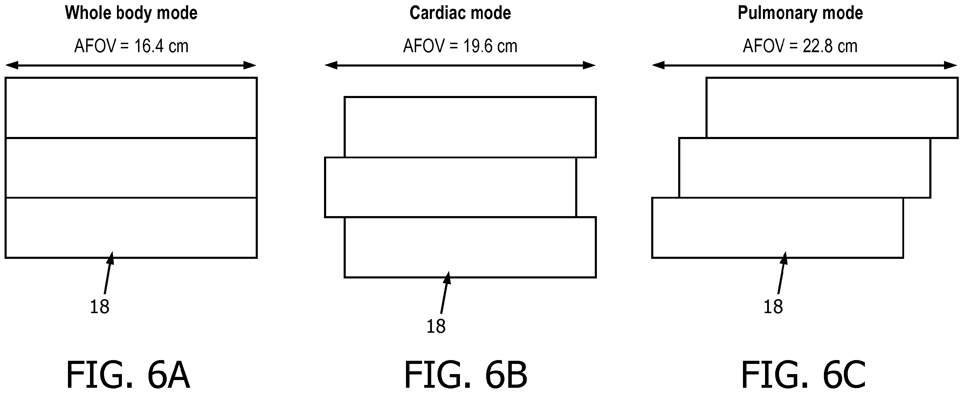

FIGURES 6A-6C show different possible configurations of the PET detector modules **18**. FIGURE 6A shows configurable PET detector modules **18** on each of the racks (not shown in FIGURE 6A) positioned next to each other, and detectors on different racks are aligned with the axial axis of the detectors. This configuration has an AFOV of 16.4 cm. The PET detector modules **18** can be moved to increase the AFOV to 19.6 cm for cardiac scans (as shown in FIGURE 6B), or to 22.8 cm (as shown in FIGURE 6C) in, for example, pulmonary or head and neck scans.

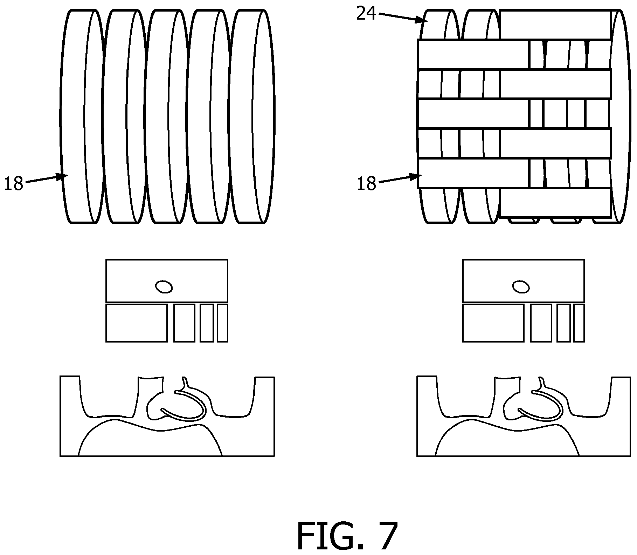

FIGURE 7 shows the PET detector modules **18** in a conventional three ring system (shown on the "left" side of FIGURE 7) can be manipulated to have the AFOV of a conventional five-ring system (as shown on the "right" side of the FIGURE 7). Data simulated from a real acquisition showed the reconfigurable system had 35% the total counts as a conventional five-ring system. The reconstructed images showed higher noise level but no degradation to the image quality otherwise.

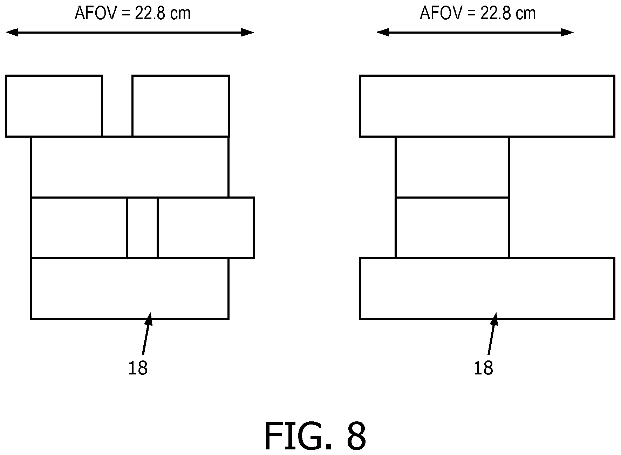

In another example, FIGURE 8 shows two other configurations of the PET detector modules **18** to extend the AFOV of the imaging system **10** from 16.4 cm to 22.8 cm. As shown on the "left" side of FIGURE 8, a gap can be formed between individual PET detector modules **18** to achieve the desired AFOV. For example, the gap can be set to 3.2 cm, and the shift in the gaps can be 1.6 cm to achieve the AFOV of 22.8 cm. As shown on the "right" side of FIGURE 8, the racks **24** can include different numbers of PET detector modules **18**. For example, the top and bottom racks **24** can include seven PET detector modules **18**, while the middle racks **24** can include four radiation detectors to achieve the AFOV of 22.8 cm.

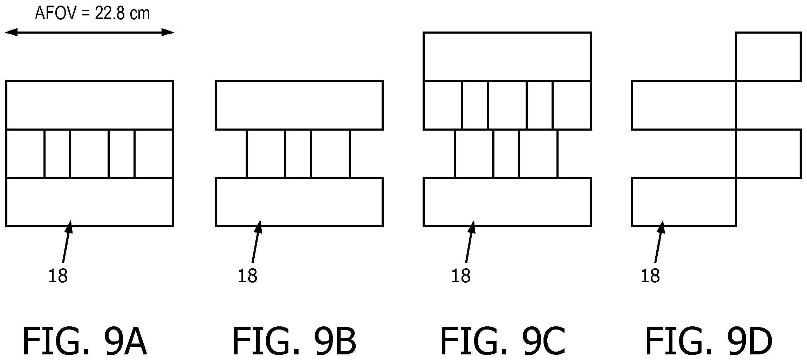

FIGURES 9A-9D show other examples of detector configurations to achieve an AFOV 22.8 cm. FIGURE 9A shows a middle rack **24** having only three PET detector modules **18** to achieve a 20% reduction of detectors to achieve the AFOV of 22.8 cm. FIGURE 9B shows a middle rack **24** having only two PET detector modules **18** to achieve a 30% reduction of detectors to achieve the AFOV of 22.8 cm. FIGURE 9C shows a first middle rack **24** having only three PET detector modules **18**, and a second middle rack having only two radiation detectors to achieve a 33% reduction of detectors to achieve the AFOV of 22.8 cm. FIGURE 9D shows alternating racks **24** having two and three PET detector modules **18** to achieve a 50% reduction of detectors to achieve the AFOV of 22.8 cm. For each of these configurations, the optimization program can reconfigure/position the PET detector modules **18** in different ways. Since the optimization program can reorient or move the PET detector modules **18** towards or away from the patient, the performance can further improved as compared to brutal force cost reduction through reducing the amount of detectors. In other words, the sensitivity decreases due to the reduction of detectors can be fully or partially compensated by the optimization program.

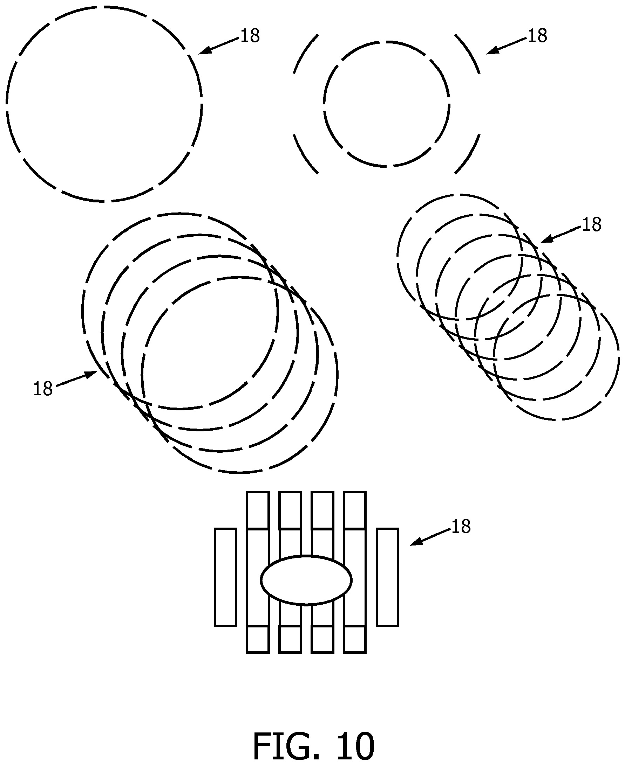

FIGURE 10 shows potential programmable configurations for imaging large and small objects. When imaging small objects, a portion of the PET detector modules **18** move in radially to get closer to the patient for better sensitivity and resolution, but can program the rest of the detectors to form additional rings or partial rings according to the configuration program, to extent the effective AFOV to further improve image sensitivity. As shown in the top left corner of FIGURE 10, a large AFOV is desired to image large objects, while a smaller a FOV is desired to image smaller objects (as shown in the central left portion of FIGURE 10). As shown in the top right corner of FIGURE 10, when imaging smaller objects in transaxial direction, the optimization program configures the PET detector modules **18** to smaller transaxial FOV rings, the extra PET detector modules **18** are programmed to form extra rings to have larger AFOV (as shown in central right and bottom portions of FIGURE 10), thus improving the imaging for small objects.

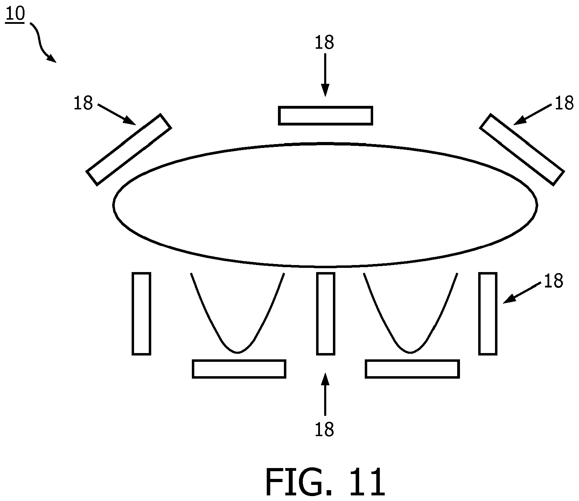

FIGURE 11 shows a PET system **10** optimized for mammography studies. As shown in FIGURE 11 shows that individual PET detector modules **18** can be positioned to image individual breasts (e.g., detectors are positioned to conform to the geometry of the breasts). Similarly, the system **10** shown in FIGURE 11 can be configured for optimized brain imaging in which a portion of the detectors can be configured to form a small rings as a conventional dedicated brain PET scanner, then some detectors can be configured to face the brain from the top of the head and some face the brain from the chin, based on the available space between patient chin and torso.

The above-described examples can be optimized based on patient size, imaging protocol, CT information, etc. to optimize the position, orientation, etc. of each PET detector module **18.** The PET detector modules **18** are positioned based on the programmed optimized position/orientation prior to or during the scans. In a first example, for a PET/CT system, a CT surview image is used to define the patient dimension and location in the imaging space. The optimization program can determine which PET detector modules **18** can be moved closer to the patient to form a ring or partial ring of smaller radius to surround the patient for optimal imaging. In a second example, a system is configured as a conventional PET/CT system with AFOV of 16.4 cm for cardiac scans. CT image (e.g., surview) shows that the patient heart has a dimension of 15 cm in the axial direction. Imaging using conventional configuration with AFOV of 16.4 will lead to significantly higher noise level near the ends of the AFOV, also correction of scatter is challenging. With the optimization program, the system can be configured to have an AFOV of 19.6 cm to allow high quality cardiac scan in one frame. Such optimization can be realized by moving the PET detector modules **18** to form a desired geometry, or by introducing gaps between PET detector modules **18** on the racks while the gap size and pattern are obtained from the optimization program. In a second example, for a conventional PET/CT system with an AFOV of 16.4 cm for cardiac scans, a CT image (e.g., surview image) shows that the patient heart has a dimension of 15 cm in the axial direction. Imaging using the conventional configuration with AFOV of 16.4 leads to significantly higher noise level near the ends of the AFOV. With the optimization program, the system can be configured to have an AFOV of 19.6 cm to allow high quality cardiac scan in one frame. Such optimization can be realized by moving the PET detector modules **18** to form a desired geometry, or by introducing gaps between radiation detectors on the racks while the gap size and pattern are obtained from the optimization program. In addition, the optimization program can include an optimization program described in co-pending Application Serial No. 62/586,229, filed November 15, 2017.

The plug-and-play configuration of the PET detector modules **18** allows for easy upgrade and maintenance, e.g., from conventional three-ring system to a five-ring system by adding two detectors on each rack in a plug-and-play model. This allows for detector sharing between scanners, maximization performance/cost for sites with multiple systems, and minimizing costs for maintenance etc.

The dynamic configuration of the PET detector modules PET detector modules **18** allows for a change to compensate for the speed ramping-up and ramping-down sensitivity change during a continue-couch-motion scan. This configuration can change during a whole-body scan to allocate better sensitivity for regions of interest, e.g. tumor area. In addition, the dynamic configuration can allow for a change during a whole-body scan to allocate less sensitivity (e.g., enlarged crystal axial distance) to less important region in the image to allow fast scans, (e.g. a leg area without a tumor). This potentially reduces the total acquisition and improve the clinical workflow and patient throughput.

The disclosure has been described with reference to the preferred embodiments. Modifications and alterations may occur to others upon reading and understanding the preceding detailed description. It is intended that the invention be construed as including all such modifications and alterations insofar as they come within the scope of the appended claims.

## Claims

1. A positron emission tomography, PET, imaging device (**10**), comprising:
a plurality of PET detector modules (**18**) arranged around a bore having a bore axis (**22**); and
a robotic gantry (**20**) operatively connected to the PET detector modules, the robotic gantry configured to control a position of each PET detector module along an axial axis (**z**), a radial axis (**r**), and a tangential axis (**θ**) of the corresponding PET detector module to move the PET detector modules (**18)** or at least groups of the PET detector modules (**18)** independently of one another in axial direction, radial direction and tangential direction,
wherein the axial axis (**z**) of the corresponding PET detector module is arranged parallel to the bore axis (**22**), the radial axis (**r**) of the corresponding PET detector module is arranged perpendicular to the bore axis (**22**), and the tangential axis (**θ**) of the corresponding PET detector module is arranged perpendicular to the bore axis (**22**) and perpendicular to the axial axis (**z**) of the corresponding PET detector module, and
wherein the plurality of PET detector modules (**18**) includes two or more axially neighboring PET detector modules and two or more tangentially neighboring PET detector modules.

2. The PET imaging device (**10**) of claim 1, wherein the robotic gantry (**20**) includes:
a plurality of racks (**24**) disposed around the bore and upon which the PET detector module (**18**) are mounted, each rack being oriented parallel with the bore axis (**22**) and each PET detector module robotically movable in the axial direction along the rack supporting the PET detector module.

3. The PET imaging device (**10**) of claim 2, wherein the robotic gantry (**20**) further includes:
telescoping robotic arms (**26**) each supporting at least one PET detector module (**18**), the telescoping robotic arms being operable to move the supported at least one PET detector module along the radial axis of the PET detector module.

4. The PET imaging device (**10**) of any one of claims 2-3, wherein the robotic gantry (**20**) further includes:
rack support arcs or rings (**28**) each at least partially encircling the bore axis (**22**), the racks (**24**) being mounted to the rack support arcs or rings by robotic links (**30**) operable to move each rack along a tangential axis transverse to the rack whereby the PET detector modules (**18**) mounted upon the rack move along the tangential axes of the corresponding PET detector module.

5. The PET imaging device (**10**) of any one of claims 1-4, further including:
a plurality of radiation shields (**32**) disposed in gaps between axially neighboring radiation detectors (**18**);
wherein the robotic gantry (**20**) is operatively connected to the radiation shields to selectively extend or retract individual radiation shields.

6. The PET imaging device (**10**) of any one of claims 1-5, wherein at least one of the PET detector modules (**18**) is different from another one of the PET detector modules, the PET detector modules being different according to at least one of:
a material used to construct the PET detector modules of the PET detector modules,
one of the PET detector modules comprising time-of-flight PET detector modules and another of the PET detector modules comprising non-time of flight PET detectors;
one of the PET detector modules comprising time-of-flight PET detector modules having a different time-of flight-resolution than another one of the PET detector modules comprising time-of-flight PET detector modules; and
one of the PET detector modules including crystals of at least one of a different size and length than crystals of another one of the PET detector modules.

7. The PET imaging device (**10**) of any one of claims 1-6, further comprising:
a robotic controller (**14**) comprising an electronic processor (**12, 14, 40**) programmed to:
determine a desired change in position along at least one of the axial axis, the radial axis, and the tangential axis of the corresponding PET detector module (**18**); and
move the corresponding PET detector module along the determined change.

8. The PET imaging device (**10**) of any one of claims 1-7, further including at least one electronic processor (**12, 14, 40**) programmed to:
control the PET detector modules (**18**) to acquire phantom or patient data in both a desired configuration and an undesired configuration of the PET detector modules;
apply a machine-learned transform to the acquired phantom or patient data to adjust the PET detector modules from the undesired configuration to the desired configuration.

9. The PET imaging device (**10**) of any one of claims 1-8, further including at least one electronic processor (**12, 14, 40**) programmed to:
determine a configuration of the PET detector modules (**18**);
acquire PET imaging data with the configuration of the PET detector modules;
model a counts distribution of the acquired imaging data using an attenuation map and a dose distribution; and
update the configuration of the radiation detectors with the counts distribution and the dose distribution.

10. The PET imaging device (**10**) of any one of claims 1-9, further including at least one electronic processor (**12, 14, 40**) programmed to:
determine a configuration of the PET detector modules (**18**) for inputs including at least one of a received imaging subject geometry and a received imaging task;
operate the robotic gantry (**20**) to arrange the plurality of PET detector modules in the determined configuration; and
with the plurality of PET detector modules arranged in the determined configuration, acquire PET imaging data including detecting coincidence events each comprising a pair of 511 keV detection events detected by PET detector modules within a coincidence time window.

11. The PET imaging device (**10**) of claim 10, wherein the at least one electronic processor (**12, 14, 40**) is programmed to determine the configuration of the PET detector modules (**18**) including axial positions of the PET detector modules to encompass the received imaging subject geometry and radial positions of the PET detector modules determined based on a girth of the received imaging subject geometry.

12. The PET imaging device (**10**) of claim 13, wherein the at least one electronic processor (**12, 14, 40**) is programmed to determine the configuration of the PET detector modules (**18**) comprising positioning of the PET detector modules conformably with at least one surface of the received imaging subject geometry.

13. The PET imaging device (**10**) of any one of claims 10-12, wherein the at least one electronic processor (**12, 14, 40**) is further programmed to:
during the acquisition of imaging data, operate the robotic gantry (**20**) to oscillate the PET detector modules (**18**) in at least one of the axial direction and the tangential directions.

14. The PET imaging device (**10**) of any one of claims 10-13, wherein the acquisition of imaging data using the PET detector modules (**18**) includes:
detecting 511 keV detection events using the PET detector modules including identifying a location of each 511 keV detection event in detector coordinates of the PET detector module;
transforming the location of each 511 keV detection event in PET detector module coordinates to a location in PET imaging device coordinates by shifting the location of the 511 keV detection event in PET detector module coordinates in accord with the position of the PET detector module along the axial axis, the radial axis, and the tangential axis of the PET detector module containing that radiation detector; and
detecting coincidence events each comprising a pair of 511 keV detection events detected by PET detector modules within a coincidence time window wherein each coincident event has an associated line of response (LOR) connecting the locations of the pair of 511 keV detection events in PET imaging device coordinates.

15. The PET imaging device (**10**) of any one of claims 10-14, wherein the at least one electronic processor (**12, 14, 40**) is programmed to repeat the determination of the detector configuration, the operating of the robotic gantry (**20**) to arrange the plurality of PET detector modules (**18**) in the determined detector configuration, and the acquisition of imaging data for a plurality of bed positions to perform multi-station imaging.

## Patentansprüche

1. Positronenemissionstomographie(PET)-Bildgebungsvorrichtung (10), umfassend:
eine Vielzahl von PET-Detektormodulen (18), die um eine Bohrung herum angeordnet sind, die eine Bohrungsachse (22) aufweist; und
eine Roboter-Gantry (20), die operativ mit den PET-Detektormodulen verbunden ist, wobei die Roboter-Gantry so konfiguriert ist, dass sie eine Position jedes PET-Detektormoduls entlang einer Axialachse (z), einer Radialachse (r) und einer Tangentialachse (θ) des entsprechenden PET-Detektormoduls steuert, um die PET-Detektormodule (18) oder zumindest Gruppen der PET-Detektormodule (18) unabhängig voneinander in axialer Richtung, radialer Richtung und tangentialer Richtung zu bewegen,
wobei die Axialachse (z) des entsprechenden PET-Detektormoduls parallel zur Bohrungsachse (22) angeordnet ist, die Radialachse (r) des entsprechenden PET-Detektormoduls senkrecht zur Bohrungsachse (22) angeordnet ist, und die Tangentialachse (θ) des entsprechenden PET-Detektormoduls senkrecht zur Bohrungsachse (22) und senkrecht zur Axialachse (z) des entsprechenden PET-Detektormoduls angeordnet ist, und
wobei die Vielzahl von PET-Detektormodulen (18) zwei oder mehr axial benachbarte PET-Detektormodule und zwei oder mehr tangential benachbarte PET-Detektormodule umfasst.

2. PET-Bildgebungsvorrichtung (10) nach Anspruch 1, wobei die Roboter-Gantry (20) umfasst:
eine Vielzahl von Zahnstangen (24), die um die Bohrung herum angeordnet sind und auf denen das PET-Detektormodul (18) montiert ist, wobei jede Zahnstange parallel zur Bohrungsachse (22) ausgerichtet ist und jedes PET-Detektormodul in axialer Richtung entlang der Zahnstange, die das PET-Detektormodul trägt, roboterbeweglich ist.

3. PET-Bildgebungsvorrichtung (10) nach Anspruch 2, wobei die Roboter-Gantry (20) ferner umfasst:
teleskopierbare Roboterarme (26), von denen jeder mindestens ein PET-Detektormodul (18) trägt, wobei die teleskopierbaren Roboterarme betreibbar sind, um das mindestens eine getragene PET-Detektormodul entlang der Radialachse des PET-Detektormoduls zu bewegen.

4. PET-Bildgebungsvorrichtung (10) nach einem der Ansprüche 2 und 3, wobei die Roboter-Gantry (20) außerdem Folgendes umfasst:
Zahnstangenstützbögen oder -ringe (28), die jeweils die Bohrungsachse (22) zumindest teilweise umkreisen, wobei die Zahnstangen (24) an den Zahnstangenstützbögen oder -ringen durch Roboterverbindungen (30) befestigt sind, die betreibbar sind, um jede Zahnstange entlang einer Tangentialachse quer zur Zahnstange zu bewegen, wobei sich die auf der Zahnstange montierten PET-Detektormodule (18) entlang der Tangentialachsen des entsprechenden PET-Detektormoduls bewegen.

5. PET-Bildgebungsvorrichtung (10) nach einem der Ansprüche 1 bis 4, ferner umfassend:
eine Vielzahl von Strahlungsabschirmungen (32), die in Lücken zwischen axial benachbarten Strahlungsdetektoren (18) angeordnet sind;
wobei die Roboter-Gantry (20) operativ mit den Strahlungsabschirmungen verbunden ist, um einzelne Strahlungsabschirmungen selektiv auszuziehen oder einzuziehen.

6. PET-Bildgebungsvorrichtung (10) nach einem der Ansprüche 1 bis 5, wobei sich mindestens eines der PET-Detektormodule (18) von einem anderen der PET-Detektormodule unterscheidet, wobei sich die PET-Detektormodule in mindestens einem der folgenden Punkte unterscheiden:
ein Material, das zum Aufbau der PET-Detektormodule der PET-Detektormodule verwendet wird,
wobei eines der PET-Detektormodule Flugzeit-PET-Detektormodule umfasst und ein anderes der PET-Detektormodule Nicht-Flugzeit-PET-Detektoren umfasst;
wobei eines der PET-Detektormodule Flugzeit-PET-Detektormodule mit einer anderen Flugzeitauflösung aufweist als ein anderes der PET-Detektormodule, das Flugzeit-PET-Detektormodule umfasst; und
wobei eines der PET-Detektormodule Kristalle mit mindestens einer anderen Größe und Länge als Kristalle eines anderen der PET-Detektormodule enthält.

7. PET-Bildgebungsvorrichtung (10) nach einem der Ansprüche 1 bis 6, ferner umfassend:
eine Robotersteuerung (14), die einen elektronischen Prozessor (12, 14, 40) umfasst, der programmiert ist zum:
Bestimmen einer gewünschten Positionsänderung entlang der Axialachse, der Radialachse und/oder der Tangentialachse des entsprechenden PET-Detektormoduls (18); und
Bewegen des entsprechende PET-Detektormoduls entlang der ermittelten Änderung.

8. PET-Bildgebungsvorrichtung (10) nach einem der Ansprüche 1 bis 7, das außerdem mindestens einen elektronischen Prozessor (12, 14, 40) umfasst, der so programmiert ist zum:
Steuern der PET-Detektormodule (18), um Phantom- oder Patientendaten sowohl in einer gewünschten Konfiguration als auch in einer unerwünschten Konfiguration der PET-Detektormodule zu erfassen;
Anwenden einer maschinell erlernten Transformation auf die erfassten Phantom- oder Patientendaten, um die PET-Detektormodule von der unerwünschten Konfiguration an die gewünschte Konfiguration anzupassen.

9. PET-Bildgebungsvorrichtung (10) nach einem der Ansprüche 1 bis 8, das außerdem mindestens einen elektronischen Prozessor (12, 14, 40) umfasst, der programmiert ist zum:
Bestimmen einer Konfiguration der PET-Detektormodule (18);
Erfassen von PET-Bilddaten mit der Konfiguration der PET-Detektormodule.
Modellieren einer Zählverteilung der erfassten Bilddaten unter Verwendung einer Schwächungskarte und einer Dosisverteilung; und
Aktualisieren der Konfiguration der Strahlungsdetektoren mit der Zählungsverteilung und der Dosisverteilung.

10. PET-Bildgebungsvorrichtung (10) nach einem der Ansprüche 1 bis 9, das außerdem mindestens einen elektronischen Prozessor (12, 14, 40) umfasst, der programmiert ist zum:
Bestimmen einer Konfiguration der PET-Detektormodule (18) für Eingaben, einschließlich mindestens einer empfangenen Bildgebungsobjektgeometrie und einer empfangenen Bildgebungsaufgabe;
Betreiben der Roboter-Gantry (20), um die mehreren PET-Detektormodule in der bestimmten Konfiguration anzuordnen; und
mit der Vielzahl von PET-Detektormodulen, die in der bestimmten Konfiguration angeordnet sind, Erfassen von PET-Bildgebungsdaten, einschließlich der Erkennung von Koinzidenzereignissen, die jeweils ein Paar von 511 keV-Erkennungsereignissen umfassen, die von PET-Detektormodulen innerhalb eines Koinzidenzzeitfensters erkannt werden.

11. PET-Bildgebungsvorrichtung (10) nach Anspruch 10, wobei der mindestens eine elektronische Prozessor (12, 14, 40) so programmiert ist, dass er die Konfiguration der PET-Detektormodule (18) einschließlich der axialen Positionen der PET-Detektormodule bestimmt, um die empfangene Bildobjektgeometrie und die radialen Positionen der PET-Detektormodule zu umfassen, die auf der Grundlage eines Umfangs der empfangenen Bildobjektgeometrie bestimmt werden.

12. PET-Bildgebungsvorrichtung (10) nach Anspruch 13, wobei der mindestens eine elektronische Prozessor (12, 14, 40) so programmiert ist, dass er die Konfiguration der PET-Detektormodule (18) bestimmt, einschließlich der Positionierung der PET-Detektormodule entsprechend mindestens einer Oberfläche der empfangenen Bildgebungsobjektgeometrie.

13. PET-Bildgebungsvorrichtung (10) nach einem der Ansprüche 10 bis 12, wobei der mindestens eine elektronische Prozessor (12, 14, 40) ferner programmiert ist zum:
während der Erfassung von Bilddaten, Betreiben der Robotergantry (20), um die PET-Detektormodule (18) in mindestens einer der axialen Richtungen und tangentialen Richtungen zu oszillieren.

14. PET-Bildgebungsvorrichtung (10) nach einem der Ansprüche 10 bis 13, wobei die Erfassung von Bildgebungsdaten unter Verwendung der PET-Detektormodule (18) Folgendes umfasst:
Erkennen von 511-keV-Erkennungsereignissen unter Verwendung der PET-Detektormodule, einschließlich Identifizieren eines Ortes jedes 511-keV-Erkennungsereignisses in Detektorkoordinaten des PET-Detektormoduls;
Transformieren des Ortes jedes 511-keV-Erkennungsereignisses in Koordinaten des PET-Detektormoduls in einen Ort in Koordinaten der PET-Bildgebungsvorrichtung durch Verschieben des Ortes des 511-keV-Erkennungsereignisses in Koordinaten des PET-Detektormoduls entsprechend der Position des PET-Detektormoduls entlang der Axialachse, der Radialachse und der Tangentialachse des PET-Detektormoduls, das diesen Strahlungsdetektor enthält; und
Erkennen von Koinzidenzereignissen, die jeweils ein Paar von 511 keV-Erkennungsereignissen umfassen, die von PET-Detektormodulen innerhalb eines Koinzidenzzeitfensters erfasst werden, wobei jedes koinzidente Ereignis eine zugehörige Reaktionslinie (LOR) aufweist, die die Orte des Paars von 511-keV-Erkennungsereignissen in den Koordinaten der PET-Bildgebungsvorrichtung verbindet.

15. PET-Bildgebungsvorrichtung (10) nach einem der Ansprüche 10 bis 14, wobei der mindestens eine elektronische Prozessor (12, 14, 40) programmiert ist, um die Bestimmung der Detektorkonfiguration und den Betrieb der Robotergantry (20) wiederzuholen, um die mehreren PET-Detektormodule (18) in der bestimmten Detektorkonfiguration anzuordnen, und Bilddaten für mehrere Bettpositionen zu erfassen, um eine Mehrstationsbildgebung durchzuführen.

## Revendications

1. Dispositif d'imagerie par tomographie par émission de positrons, TEP, (10) comprenant:
une pluralité de modules détecteurs TEP (18) disposés autour d'un alésage ayant un axe d'alésage (22); et
un portique robotique (20) connecté de manière opérative aux modules détecteurs TEP, le portique robotique étant configuré pour commander une position de chaque module détecteur TEP le long d'un axe axial (z), d'un axe radial (r), et d'un axe tangentiel (θ) du module détecteur TEP correspondant pour déplacer les modules détecteurs TEP (18) ou au moins des groupes de modules détecteurs TEP (18) indépendamment les uns des autres dans la direction axiale, la direction radiale et la direction tangentielle,
où l'axe axial (z) du module détecteur TEP correspondant est disposé parallèlement à l'axe d'alésage (22), l'axe radial (r) du module détecteur TEP correspondant est disposé perpendiculairement à l'axe d'alésage (22), et l'axe tangentiel (θ) du module détecteur TEP correspondant est disposé perpendiculairement à l'axe d'alésage (22) et perpendiculairement à l'axe axial (z) du module détecteur TEP correspondant, et
où la pluralité de modules détecteurs TEP (18) comprend deux ou plusieurs modules détecteurs TEP axialement voisins et deux ou plusieurs modules détecteurs TEP tangentiellement voisins.

2. Dispositif d'imagerie TEP (10) selon la revendication 1, dans lequel le portique robotique (20) comprend:
une pluralité de crémaillères (24) disposées autour de l'alésage et sur lesquelles le module détecteur TEP (18) est monté, chaque crémaillère étant orientée parallèlement à l'axe de l'alésage (22) et chaque module détecteur TEP se déplaçant robotiquement dans la direction axiale le long de la crémaillère supportant le module détecteur TEP.

3. Dispositif d'imagerie TEP (10) selon la revendication 2, dans lequel le portique robotique (20) comprend en outre:
des bras robotiques télescopiques (26) supportant chacun au moins un module détecteur TEP (18), les bras robotiques télescopiques pouvant être actionnés pour déplacer au moins un module détecteur TEP supporté le long de l'axe radial du module détecteur TEP.

4. Dispositif d'imagerie TEP (10) selon l'une quelconque des revendications 2 et 3, dans lequel le portique robotique (20) comprend en outre:
des arcs ou des anneaux de support de crémaillère (28) encerclant chacun au moins partiellement l'axe d'alésage (22), les crémaillères (24) étant montées sur les arcs ou anneaux de support de crémaillère par des liaisons robotiques (30) pouvant être actionnées pour déplacer chaque crémaillère le long d'un axe tangentiel transversal à la crémaillère, ce qui permet aux modules détecteurs TEP (18) montés sur la crémaillère de se déplacer le long des axes tangentiels du module détecteur TEP correspondant.

5. Dispositif d'imagerie TEP (10) selon l'une quelconque des revendications 1 à 4, comprenant en outre:
une pluralité d'écrans anti-rayonnement (32) disposés dans des espaces entre des détecteurs de rayonnement axialement voisins (18);
où le portique robotique (20) est connecté de manière opérative aux écrans anti-rayonnement pour étendre ou rétracter sélectivement les écrans anti-rayonnement individuels.

6. Dispositif d'imagerie TEP (10) selon l'une quelconque des revendications 1 à 5, dans lequel au moins un des modules détecteurs TEP (18) est différent d'un autre des modules détecteurs TEP, les modules détecteurs TEP étant différents selon au moins l'un parmi :
un matériau utilisé pour construire les modules détecteurs TEP des modules détecteurs TEP,
un des modules détecteurs TEP comprenant des modules détecteurs TEP à temps de vol et un autre des modules détecteurs TEP comprenant des détecteurs TEP à temps de vol;
un des modules détecteurs TEP comprenant des modules détecteurs TEP à temps de vol ayant une résolution à temps de vol différente de celle d'un autre des modules détecteurs TEP comprenant des modules détecteurs TEP à temps de vol; et
un des modules détecteurs TEP comprenant des cristaux d'au moins une taille et une longueur différentes de celles des cristaux d'un autre des modules détecteurs TEP.

7. Dispositif d'imagerie TEP (10) selon l'une quelconque des revendications 1 à 6, comprenant en outre:
un dispositif de commande robotique (14) comprenant un processeur électronique (12, 14, 40) programmé pour:
déterminer un changement de position souhaité le long d'au moins l'un parmi l'axe axial, l'axe radial, et l'axe tangentiel du module détecteur TEP correspondant (18); et
déplacer le module détecteur TEP correspondant le long du changement déterminé.

8. Dispositif d'imagerie TEP (10) selon l'une quelconque des revendications 1 à 7, comprenant en outre au moins un processeur électronique (12, 14, 40) programmé pour:
commander les modules détecteurs TEP (18) pour acquérir des données fantômes ou de patient à la fois dans une configuration souhaitée et dans une configuration non souhaitée des modules détecteurs TEP;
appliquer une transformation apprise par machine aux données fantômes ou du patient acquises pour ajuster les modules détecteurs TEP de la configuration non souhaitée à la configuration souhaitée.

9. Dispositif d'imagerie TEP (10) selon l'une quelconque des revendications 1 à 8, comprenant en outre au moins un processeur électronique (12, 14, 40) programmé pour:
déterminer une configuration des modules détecteurs TEP (18);
acquérir des données d'imagerie TEP avec la configuration des modules détecteurs TEP;
modéliser une distribution de comptages des données d'imagerie acquises à l'aide d'une carte d'atténuation et d'une distribution de dose; et
mettre à jour la configuration des détecteurs de rayonnement avec la distribution des comptages et la distribution de dose.

10. Dispositif d'imagerie TEP (10) selon l'une quelconque des revendications 1 à 9, comprenant en outre au moins un processeur électronique (12, 14, 40) programmé pour:
déterminer une configuration des modules détecteurs TEP (18) pour des entrées comprenant au moins l'une entre une géométrie de sujet d'imagerie reçue et une tâche d'imagerie reçue;
faire fonctionner le portique robotique (20) pour agencer la pluralité de modules détecteurs TEP dans la configuration déterminée; et
avec la pluralité de modules détecteurs TEP agencés dans la configuration déterminée, acquérir des données d'imagerie TEP comprenant la détection d'événements de coïncidence comprenant chacun une paire d'événements de détection de 511 keV détectés par des modules détecteurs TEP dans une fenêtre temporelle de coïncidence.

11. Dispositif d'imagerie TEP (10) selon la revendication 10, dans lequel l'au moins un processeur électronique (12, 14, 40) est programmé pour déterminer la configuration des modules détecteurs TEP (18), y compris les positions axiales des modules détecteurs TEP pour englober la géométrie du sujet d'imagerie reçue et les positions radiales des modules détecteurs TEP déterminées sur la base d'une circonférence de la géométrie du sujet d'imagerie reçue.

12. Dispositif d'imagerie TEP (10) selon la revendication 13, dans lequel l'au moins un processeur électronique (12, 14, 40) est programmé pour déterminer la configuration des modules détecteurs TEP (18) comprenant le positionnement des modules détecteurs TEP de manière conforme à au moins une surface de la géométrie du sujet d'imagerie reçue.

13. Dispositif d'imagerie TEP (10) selon l'une quelconque des revendications 10 à 12, dans lequel l'au moins un processeur électronique (12, 14, 40) est en outre programmé pour:
pendant l'acquisition de données d'imagerie, faire fonctionner le portique robotique (20) pour faire osciller les modules détecteurs TEP (18) dans au moins l'une entre la direction axiale et les directions tangentielles.

14. Dispositif d'imagerie TEP (10) selon l'une quelconque des revendications 10 à 13, dans lequel l'acquisition de données d'imagerie à l'aide des modules détecteurs TEP (18) comprend:
la détection d'événements de détection de 511 keV à l'aide des modules détecteurs TEP, y compris l'identification d'un emplacement de chaque événement de détection de 511 keV dans les coordonnées de détecteur du module détecteur TEP;
la transformation de l'emplacement de chaque événement de détection de 511 keV dans les coordonnées du module détecteur TEP en un emplacement dans les coordonnées du dispositif d'imagerie TEP en décalant l'emplacement de l'événement de détection de 511 keV dans les coordonnées du module détecteur TEP en accord avec la position du module détecteur TEP le long de l'axe axial, l'axe radial, et l'axe tangentiel du module détecteur TEP contenant ce détecteur de rayonnement; et
la détection d'événements de coïncidence comprenant chacun une paire d'événements de détection de 511 keV détectés par des modules détecteurs TEP dans une fenêtre temporelle de coïncidence où chaque événement coïncident a une ligne de réponse (LOR) associée reliant les emplacements de la paire d'événements de détection de 511 keV dans les coordonnées d'un dispositif d'imagerie TEP.

15. Dispositif d'imagerie TEP (10) selon l'une quelconque des revendications 10 à 14, dans lequel l'au moins un processeur électronique (12, 14, 40) est programmé pour répéter la détermination de la configuration du détecteur, le fonctionnement du portique robotique (20) pour agencer la pluralité de modules détecteurs TEP (18) dans la configuration de détecteur déterminée, et l'acquisition de données d'imagerie pour une pluralité de positions de lit afin d'effectuer une imagerie multi-stations.
